# FASCICULE DE BREVET EUROPEEN

(11) **EP 0 479 627 B1**
(45) Date de publication et mention de la délivrance du brevet: **10.07.1996**
(21) Numéro de dépôt: 91400513.7
(22) Date de dépôt: 26.02.1991
(51) Int. Cl.: A61K 39/00, A61K 39/245

(54) **Association vaccinale contre les pathogènes infectieux**
Vakzinale Zusammensetzung gegen infektiöse Pathogene
Vaccinal association against infections pathogens

(30) Priorité: 01.10.1990 FR 9012073
(43) Date de publication de la demande: 08.04.1992
(73) Titulaire: RHONE MERIEUX S.A., 69002 Lyon (FR)
(72) Inventeur: Biront, Patrick, B-3050 Oud-Heverlee (BE); Pensaert, Maurice, B-9290 Berlare (BE); Vandeputte, Joris, F-38790 Diemoz (FR)
(74) Mandataire: Bernasconi, Jean

(56) Documents cités:
- EP-A- 0 353 809
- JOURNAL OF IMMUNOLOGY, vol. 119, no. 6, Décembre 1977, BALTIMORE US, pages 2073-2077; R.G. WEBSTER ET AL: "POTENTIATION OF THE IMMUNE RESPONSE TO INFLUENZA VIRUS SUBUNIT VACCINES"
- F. FENNER ET AL. "VETERINARY VIROLOGY" 1987, ACADEMIC PRESS, INC., ORLANDO, FLA, US; p.272/

## Description

La présente invention concerne de nouvelles associations vaccinales contre les pathogènes infectieux viraux, bactériens et parasitaires.

D'une façon générale, dans le domaine de la préparation des vaccins humains et vétérinaires, on cherche à réaliser des vaccins purifiés, dits de sous-unités, composés d'un immunogène ou des immunogènes de même nature d'un agent pathogène infectieux, ou même uniquement du déterminant antigénique de l'immunogène, afin d'obtenir une réponse immunitaire protectrice bien définie et, également, d'éliminer des constituants mal connus ou toxiques.

Quant aux associations vaccinales, elles cherchent, généralement, à réunir des valences de pathogènes hétérologues, ou pour le moins de sérotypes différents, de façon à élargir le spectre protecteur, cette démarche s'étendant aux vaccins vivants recombinants.

Certes, dans quelques cas isolés, on a suggéré de potentialiser un vaccin de sous-unités par l'administration simultanée d'une faible quantité du pathogène entier.

W.G. LAVER et R.G. WEBSTER (Virology 69, 511-522, 1976) ont démontré, pour le virus de la grippe chez le hamster et la souris, la potentialisation de la réponse immunitaire par l'injection simultanée du virus entier intact et de vaccin sous-unités neuraminidase-hémagglutinine ou par l'injection du virus intact une heure avant celle de ce vaccin sous-unités.

R.G. WEBSTER et al. (The Journal of Immunology, vol. 118, N° 6, Décembre 1977, p. 2073 à 2077) ont ensuite montré, chez le hamster et l'homme, l'effet potentialisateur de l'addition de virus de la grippe inactivé, au vaccin sous-unités neuraminidase-hémagglutinine.

Il s'agit, là, de virus de la grippe inactivé entier, et la quantité de pathogène entier est faible et destinée à potentialiser l'effet protecteur du vaccin de sous-unités.

Or la présente invention a révélé, de façon surprenante, dans le cas de pathogènes très éloignés du virus de la grippe, tels que notamment les herpèsviridae, que l'association d'un vaccin de sous-unités d'enveloppe ou paroi, c'est-à-dire d'immunogènes immédiatement apparents ou facilement apparents du même pathogène, améliore grandement l'efficacité de la vaccination, et que cette propriété s'étend à de nombreux pathogènes, y compris dans des groupes aussi différents que les virus, les bactéries et les parasites, notamment protozoaires.

L'invention se propose d'améliorer de façon significative l'efficacité de la vaccination contre les maladies infectieuses des animaux ou de l'homme.

La présente invention a donc pour objet une association vaccinale comprenant une première valence comportant un agent microbien à l'état vivant atténué ou un recombinant exprimant des protéines ou glycoprotéines antigéniques dudit agent, et une deuxième valence comportant une ou des glycoprotéines d'enveloppe ou de paroi antigéniques ou une protéine structurale antigénique, notamment du même sérotype.

Par agent microbien dans le sens de la présente invention, on entend un virus, une bactérie ou un parasite appartenant à une espèce pathogène et pour lequel il existe déjà, ou l'on peut produire, une forme vivante ou inactivée (délétée ou non) immunogène et, au moins partiellement, protectrice, ou bien encore pour lequel il existe, ou l'on peut construire, des séquences nucléotidiques insérables dans un micro-organisme hétérologue recombinant utilisable comme un vaccin et exprimant des protéines ou des glycoprotéines antigéniques au moins partiellement protectrices codées par une telle séquence.

Parmi les agents viraux, on peut citer notamment, outre les virus herpès, les parvovirus, les coronavirus, les virus de la grippe humaine, porcine, équine, le virus de la poliomyélite, les virus de la rage.

Parmi les bactéries, on peut citer notamment les Brucella.

Parmi les micro-organismes hétérologues, on peut citer notamment les pox-virus, par exemple la vaccine ou les pox-virus aviaires, et les virus herpès.

On doit comprendre que, dans la présente invention, la deuxième valence provient généralement du même sérotype (lorsque des sérotypes différents existent) que l'agent fournissant la première valence, mais que, dans certains cas, les sérotypes peuvent différer lorsqu'il est établi qu'une protection croisée efficace existe entre les sérotypes.

La présente invention a notamment pour objet une association vaccinale herpétique humaine ou animale comprenant une première valence comportant le virus herpétique considéré à l'état vivant atténué (délété ou non délété) ou un recombinant exprimant des glycoprotéines antigéniques dudit virus et une deuxième valence comportant une ou des glycoprotéines d'enveloppe antigéniques notamment du même sérotype et éventuellement d'autres éléments antigéniques dudit virus.

Parmi les herpès animaux, l'invention concerne notamment les herpès virus porcin, équin, bovin, félin et aviaires.

Les valences peuvent être constituées de vaccins déjà connus destinés à l'homme ou aux animaux. Ainsi, on connaît notamment des vaccins à base de virus vivant atténué ou des vaccins à base de sous-unités virales, notamment de glycoprotéines d'enveloppe et de nucléocapsides. Pour la maladie d'Aujeszky, on peut citer par exemple le vaccin Geskalone® de Rhône Mérieux, France, qui est un vaccin vivant lyophilisé atténué en solvant aqueux ou huileux, et le vaccin Geskypur®, également de Rhône Mérieux, qui est un vaccin de sous-unités, liquide ou lyophilisé, avec adjuvant, constitué de glycoprotéines d'enveloppe purifiées.

Selon l'invention, cette association vaccinale peut se présenter sous la forme d'une préparation unique, d'un mélange extemporané ou avec les deux valences séparées pour une administration simultanée séparée. Par administration simultanée séparée, on entend des injections en des points différents de l'organisme, dans un intervalle de temps réduit, ne dépassant pas quelques heures et, de préférence, quelques minutes.

Dans le cas d'une préparation prête à l'emploi, la valence à glycoprotéines et la valence de l'agent ou virus atténué ou de recombinant sont en solution dans un solvant aqueux ou huileux.

De préférence, dans les autres cas, la valence atténuée ou de recombinant se présente sous forme lyophilisée et la valence à glycoprotéines, sous forme liquide ou lyophilisée.

Les deux valences peuvent se trouver soit à l'état lyophilisé et en mélange dans un même flacon pour être ensuite reprises simultanément avec un solvant aqueux ou huileux, soit la première valence peut être reprise avec la deuxième valence initialement à l'état liquide en milieu aqueux ou huileux ou encore chacune des valences, lyophilisées et séparées, peuvent être reprises avec un solvant de même nature, aqueux ou huileux, puis mélangées.

Dans le cas d'une administration simultanée séparée, les solvants des valences peuvent être aqueux, huileux ou respectivement aqueux et huileux.

L'association vaccinale sera de préférence présentée dans un emballage unique.

Dans le cas d'une préparation unique, l'association vaccinale est présentée prête à l'emploi. Son administration est notamment intramusculaire ou intradermique.

Dans le cas d'un mélange extemporané, les deux valences sont à l'état lyophilisé dans un même flacon et doivent être reprises avec un solvant aqueux ou huileux, ou chaque valence est présentée séparément et les deux valences doivent donc être réunies au moment de l'administration, de la manière décrite ci-dessus. Le mode d'administration est notamment intramusculaire ou intradermique.

Dans le cas d'une administration simultanée séparée, les deux valences sont présentées séparément. La valence à glycoprotéines peut être prête à l'emploi ou, comme la première valence, peut se présenter sous forme lyophilisée et doit donc être mise en solution avant utilisation. Le mode d'administration est notamment intramusculaire ou intradermique pour la valence comportant l'agent ou virus atténué ou un recombinant tandis que, pour l'autre, le mode d'administration peut être intradermique, intramusculaire ou sous-cutané.

Comme association vétérinaire herpétique, on peut citer par exemple l'association Geskalone®-Geskypur®.

Il va de soi que l'association selon l'invention concerne tous les vaccins atténués ou à sous-unités à glycoprotéines connus. Pour les herpès équin, bovin, félin et aviaire (y compris laryngotrachéite), on connaît déjà des vaccins vivants atténués et l'on peut citer, à titre d'exemple, les vaccins sous-unités suivants :
- équin : PNEUMEQUINE® (Rhône Mérieux), vaccin de la rhinopneumonie équine ;
- bovin : IBEPUR® (Rhône Mérieux), vaccin de la rhinotrachéite infectieuse des bovins ;
- félin : CORIFELIN® ou LEUCORIFELIN® (Rhône Mérieux), vaccin du coryza ;
- aviaire : laryngotrachéite.

L'invention a aussi pour objet un procédé de vaccination, notamment contre les herpès-virus humains ou animaux, comprenant l'administration simultanée des deux valences décrites ci-dessus, mélangées ou séparées, par le mode d'administration approprié mentionné ci-dessus.

L'invention va être maintenant décrite plus en détail à l'aide d'exemples de réalisation et d'essai in vivo.

### I. ESSAI

On a vacciné 18 porcs contre la maladie d'Aujeszky. Ces porcs provenaient de truies vaccinées avec le vaccin TOLVID (vaccin atténué délété pour gX) (deux fois par an). Le protocole a été le suivant :
- 6 porcs non vaccinés servent de témoins,
- 6 porcs sont vaccinés une fois avec la souche Bartha reprise dans un adjuvant de Solvay huile en eau, le vaccin titrant 10^{5,5} CCID.50 par dose,
- 6 porcs sont vaccinés une fois avec une dose de GESKALONE gI-, titrant 10^{5,5} CCID.50 par dose, repris dans 2 ml de GESKYPUR.

Tous ces porcs sont stabulés ensemble et 4 semaines après la vaccination, ils ont tous été éprouvés par voie oronasale avec 10^{5,5} CCID.50, cette dose ayant été administrée trois fois durant 24 heures.

### Résultats sérologiques (anticorps séroneutralisants) :

- les animaux témoins restent négatifs jusqu'à l'épreuve : titre < 2,
- les animaux vaccinés avec Bartha ont des titres moyens au moment de l'épreuve variant de 4 à 16,
- les animaux vaccinés GESKYPUR/GESKALONE :
   . 1 animal : titre de 4
   . 1 animal : titre de 32
   . 4 animaux ont des titres de 96

### Epreuve :

Paramètre de l'excrétion virale

| | Durée | Titre | Nombre d'animaux excrétant |
|---|---|---|---|
| témoins | 5/6 jours | - | tous |
| Bartha | 2/3 jours | 10^{2 à 3} | 4 sur 6 |
| GESKYPUR | 1 jour | à peine | 2 sur 6 |
| /GESKALONE | | détectable | |

On obtient donc des titres sérologiques et des résultats d'épreuve exceptionnels.

### II. Exemples

1/ On réalise une préparation unique contenant une dose de Geskalone reprise avec 2 ml de Geskypur. Cette préparation est prête à l'emploi et peut être administrée par voie intramusculaire ou intradermique.
2/ On dispose, dans un emballage unique, d'une dose de Geskalone lyophilisée et d'une dose de Geskypur prête à l'emploi. Au moment de l'administration, on reprend le Geskalone avec le Geskypur. L'administration s'effectue par voie intramusculaire ou intradermique.
3/ Idem 2/, mais on reprend le Geskalone avec un solvant approprié, aqueux ou huileux, et on administre les deux valences de façon simultanée et séparée, par voie intradermique ou intramusculaire, ou encore sous-cutanée pour le Geskypur.

Par voie intradermique, les deux volumes de dose sont de préférence de 0,2 ml pour chaque valence ou pour les deux valences réunies.

Pour les porcs reproducteurs, on prévoit par exemple une primo-vaccination avec 2 injections à 3 ou 4 semaines d'intervalle et des rappels tous les six mois.

## Revendications (Revendications pour l'(les) Etat(s) contractant(s) suivant(s): AT, BE, CH, DE, DK, FR, GB, IT, LT, LU, NL, SE)

1. Association vaccinale contre une maladie infectieuse, caractérisée en ce qu'elle comprend une première valence comportant un agent microbien à l'état vivant atténué ou un recombinant exprimant des protéines ou glycoprotéines antigéniques dudit agent, et une deuxième valence comportant une ou des glycoprotéines d'enveloppe ou de paroi antigéniques ou une protéine structurale antigénique, notamment du même sérotype.

2. Association selon la revendication 1, caractérisée en ce que l'agent est choisi parmi les virus les bactéries et les parasites.

3. Association selon la revendication 2, caractérisée en ce que l'agent est choisi parmi ceux pour lesquels on connaît une forme vivante atténuée au moins partiellement protectrice ou un recombinant au moins partiellement protecteur.

4. Association vaccinale selon l'une quelconque des revendications 2 et 3 dirigée contre un virus de la famille des herpès-virus animaux ou humains et comprenant, dans une préparation unique, une première valence comportant ledit virus à l'état vivant atténué ou un recombinant exprimant des glycoprotéines antigéniques dudit virus et une deuxième valence comportant des glycoprotéines d'enveloppe antigéniques dudit virus.

5. Association vaccinale selon la revendication 4, caractérisée en ce que les deux valences sont mélangées de façon extemporanée.

6. Association vaccinale selon l'une quelconque des revendications 2 et 3 dirigée contre un virus de la famille des herpès-virus animaux ou humains et comprenant séparément, pour l'administration simultanée, d'une part une première valence comportant ledit virus à l'état vivant atténué ou un recombinant exprimant des glycoprotéines antigéniques dudit virus et d'autre part une deuxième valence comportant des glycoprotéines d'enveloppe antigéniques dudit virus.

7. Association vaccinale prête à l'emploi selon la revendication 4, caractérisée en ce que les deux valences sont en solution dans un solvant aqueux ou huileux.

8. Association vaccinale selon la revendication 5, caractérisée en ce que les deux valences se présentent sous forme lyophilisée et en mélange pour être reprises simultanément avec un solvant huileux ou aqueux.

9. Association vaccinale selon la revendication 5, caractérisée en ce que la première valence est lyophilisée et la deuxième, liquide, la première devant être reprise avec la deuxième.

10. Association vaccinale selon la revendication 5, caractérisée en ce que chaque valence est lyophilisée et doit être reprise avec un solvant, de même nature, aqueux ou huileux puis mélangée avec l'autre valence.

11. Association vaccinale selon la revendication 6, caractérisée en ce que les deux valences sont lyophilisées et doivent être reprises séparément avec un solvant aqueux ou huileux.

12. Association vaccinale selon la revendication 6, caractérisée en ce que la deuxième valence est liquide et prête à l'emploi, tandis que !a première est lyophilisée et doit être reprise avec un solvant aqueux ou huileux.

13. Association vaccinale selon l'une quelconque des revendications 4 à 12, caractérisée en ce que le virus animal concerné est un virus herpétique porcin, équin, bovin, félin ou aviaire.

14. Association vaccinale selon la revendication 13, caractérisée en ce que le virus concerné est le virus de la maladie d'Aujeszky.

15. Association vaccinale selon l'une quelconque des revendications 1 à 14, caractérisée en ce qu'elle est présentée dans un emballage unique.

## Revendications (Revendications pour l'(les) Etat(s) contractant(s) suivant(s): ES, GR)

1. Procédé de réalisation d'un vaccin contre une maladie infectieuse, caractérisé en ce qu'on conditionne dans un emballage unique une première valence comportant un agent microbien à l'état vivant atténué ou un recombinant exprimant des protéines ou glycoprotéines antigéniques dudit agent, et une deuxième valence comportant une ou des glycoprotéines d'enveloppe ou de paroi antigéniques ou une protéine structurale antigénique, notamment du même sérotype.

2. Procédé selon la revendication 1, caractérisé en ce que l'agent est choisi parmi les virus, les bactéries et les parasites.

3. Procédé selon la revendication 2, caractérisé en ce que l'agent est choisi parmi ceux pour lesquels on connaît une forme vivante atténuée au moins partiellement protectrice ou un recombinant au moins partiellement protecteur.

4. Procédé selon l'une quelconque des revendications 2 et 3 pour la réalisation d'un vaccin dirigé contre un virus de la famille des herpès-virus animaux ou humains, caractérisé en ce qu'on conditionne dans un emballage unique, une première valence comportant ledit virus à l'état vivant atténué ou un recombinant exprimant des glycoprotéines antigéniques dudit virus et une deuxième valence comportant des glycoprotéines d'enveloppe antigéniques dudit virus.

5. Procédé selon la revendication 4, caractérisé en ce que les deux valences sont mélangées.

6. Procédé selon la revendication 5, caractérisé en ce que les deux valences sont en solution dans un solvant aqueux ou huileux.

7. Procédé selon la revendication 4, caractérisé en ce que l'on conditionne les deux valences sous forme lyophilisée et en mélange de façon qu'elles puissent être reprises de façon extemporanée et simultanée avec un solvant huileux ou aqueux.

8. Procédé selon la revendication 4, caractérisé en ce qu'on conditionne la première valence sous forme lyophilisée et la deuxième sous forme liquide, dans un solvant apte à reprendre la première valence.

9. Procédé selon la revendication 4, caractérisé en ce qu'on conditionne chaque valence sous une forme lyophilisée pouvant être reprise avec un solvant, de même nature pour chacune des valences, aqueux ou huileux, puis mélangée avec l'autre valence.

10. Procédé selon la revendication 4, caractérisé en ce que les deux valences sont conditionnées chacune sous une forme lyophilisée de façon à pouvoir être reprises séparément avec un solvant aqueux ou huileux.

11. Procédé selon la revendication 4, caractérisé en ce qu'on conditionne la deuxième valence sous forme liquide et prête à l'emploi, tandis qu'on conditionne la première sous une forme lyophilisée devant être reprise avec un solvant aqueux ou huileux.

12. Procédé selon l'une quelconque des revendications 4 à 11, caractérisé en ce que le virus animal concerné est un virus herpétique porcin, équin, bovin, félin ou aviaire.

13. Procédé selon la revendication 12, caractérisé en ce que le virus concerné est le virus de la maladie d'Aujeszky.

## Claims (Claims for the following Contracting State(s): AT, BE, CH, DE, DK, FR, GB, IT, LT, LU, NL, SE)

1. Vaccine association against an infectious disease, characterized in that it comprises a first valence containing a microbial agent in live attenuated state or a recombinant expressing antigenic proteins or glycoproteins of said agent, and a second valence containing one or some antigenic glycoproteins of the envelope or wall or an antigenic structural protein, in particular of the same zero-type.

2. Association according to Claim 1, characterised in that the agent is selected from viruses, bacteria and parasites.

3. Association according to Claim 2, characterised in that the agent is selected from those for which an at least partially protective live attenuated form or an at least partially protective recombinant is known.

4. Vaccine association according to any one of Claims 2 and 3 directed against a virus of the animal or human herpes virus family and containing in a single preparation a first valence comprising said virus in live attenuated state or a recombinant expressing the antigenic glycoproteins of said virus and a second valence comprising the antigenic glycoproteins of the envelope of said virus.

5. Vaccine association according to Claim 4, characterised in that the two valences are mixed in an extemporaneous manner.

6. Vaccine association according to any one of Claims 2 and 3 directed against a virus of the animal or human herpes virus family and separately containing for simultaneous administration a first valence containing said virus in live attenuated state or a recombinant expressing the antigenic glycoproteins of said virus, on the one hand, and a second valence containing the antigenic glycoproteins of the envelope of said virus, on the other hand.

7. Vaccine association ready for use according to Claim 4, characterised in that two valences are in solution in an aqueous or oil solvent.

8. Vaccine association according to Claim 5, characterized in that the two valences are present in lyophilised form and in mixture to be incorporated simultaneously into an oil or aqueous solvent.

9. Vaccine association according to Claim 5, characterised in that the first valence is lyophilised and the second is liquid, the first having to be incorporated into the second.

10. Vaccine association according to Claim 5, characterised in that each valence is lyophilised and must be incorporated into a solvent of the same type, aqueous or oil, then mixed with the other valence.

11. Vaccine association according to Claim 6, characterised in that the two valences are lyophilised and must be incorporated separately into an aqueous or oil solvent.

12. Vaccine association according to Claim 6, characterised in that the second valence is liquid and ready for use, whereas the first is lyophilized and must be incorporated into an aqueous or oil solvent.

13. Vaccine association according to any one of Claims 4 to 12, characterised in that the animal virus is a porcine, equine, bovine, feline or fowl herpes virus.

14. Vaccine association according to Claim 13, characterised in that the virus concerned is the Aujeszky disease virus.

15. Vaccine association according to any one of Claims 1 to 14, characterised in that it is presented in a single package.

## Claims (Claims for the following Contracting State(s): ES, GR)

1. Process for producing a vaccine against an infectious disease, characterized in that a first valence containing a microbial agent in live attenuated state or a recombinant expressing antigenic proteins or glycoproteins of said agent, and a second valence containing one or some antigenic glycoproteins of the envelope or wall or an antigenic structural protein, in particular of the same sero-type, are contained in a single preparation.

2. Process according to Claim 1, characterised in that the agent is selected from viruses, bacteria and parasites.

3. Process according to Claim 2, characterized in that the agent is selected from those for which an at least partially protective live attenuated form or an at least partially protective recombinant is known.

4. Process according to any one of Claims 2 and 3 directed against a virus of the animal or human herpes virus family, characterised in that a first valence comprising said virus in live attenuated state or a recombinant expressing the antigenic glycoproteins of said virus and a second valence comprising the antigenic glycoproteins of the envelope of said virus are contained in a single preparation.

5. Process according to Claim 4, characterised in that the two valences are mixed.

6. Process according to any one of Claim 5, characterised in that two valences are in solution in an aqueous or oil solvent.

7. Process according to Claim 4, characterised in that the two valences are prepared in lyophilised form and in mixture so that they may be incorporated extemporaneously and simultaneously into an oil or aqueous solvent.

8. Process according to Claim 4, characterised in that the first valence is prepared in lyophilised form and the second in liquid form in a solvent able to incorporate the first valence.

9. Process according to Claim 4, characterised in that each valence is prepared in lyophilised form able to be incorporated into a solvent of the same type for each valence, either aqueous or oil, then mixed with the other valence.

10. Process according to Claim 4, characterised in that the two valences are each prepared in a lyophilised form so that they may be incorporated separately into an aqueous or oil solvent.

11. Process according to Claim 4, characterised in that the second valence is prepared in liquid form and ready for use, whereas the first is prepared in lyophilised form which must be incorporated into an aqueous or oil solvent.

12. Process according to any one of Claims 4 to 11, characterised in that the animal virus is a porcine, equine, bovine, feline or fowl herpes virus.

13. Vaccine association according to Claim 12, characterised in that the virus concerned is the Aujeszky disease virus.

## Patentansprüche (Patentansprüche für folgende(n) Vertragsstaat(en): AT, BE, CH, DE, DK, FR, GB, IT, LT, LU, NL, SE)

1. Polyvalentes Vakzin gegen eine infektiöse Krankheit, dadurch gekennzeichnet, daß sie eine erste Valenz umfaßt, die ein mikrobielles Mittel im lebend attenuierten Zustand oder einen Rekombinanten, welcher antigene Proteine oder Glykoproteine dieses Mittels exprimiert, enthält und eine zweite Valenz umfaßt, die ein oder mehrere antigene Hüllenglykoproteine oder Wände oder antigene Strukturproteine, insbesondere desselben Serotyps enthält.

2. Polyvalentes Vakzin nach Anspruch 1, daß das Mittel ausgewählt ist aus den Viren, den Bakterien und den Parasiten.

3. Polyvalentes Vakzin nach Anspruch 2, dadurch gekennzeichnet, daß das Mittel von denjenigen ausgewählt ist, von denen man eine wenigstens teilweise schützende lebend attenuierte Form oder einen wenigstens teilweise schützenden Rekombinanten kennt.

4. Polyvalentes Vakzin nach einem der Ansprüche 2 oder 3, die gegen einen Virus aus der Familie der tierischen oder menschlichen Herpesviren gerichtet ist und in einer einzigen Zubereitung eine erste Valenz umfaßt, die diesen Virus im lebend attenuierten Zustand oder einen Rekombinanten, welcher antigene Glykoproteine dieses Virus exprimiert, enthält und eine zeite Valenz unfaßt, die antigene Hüllenglykoproteine dieses Virus enthält.

5. Polyvalentes Vakzin nach Anspruch 4, dadurch gekennzeichnet, daß die beiden Valenzen unmittelbar vor Anwendung vermischt werden.

6. Polyvalentes Vakzin nach einem der Ansprüche 2 und 3, die gegen einen Virus aus der Familie der tierischen oder menschlichen Herpesviren gerichtet ist und getrennt, zur gleichzeitigen Verabreichung einerseite eine erste Valenz umfaßt, die diesen Virus im lebend attenuierten Zustand oder einen Rekombinanten, der ein antigenes Glykoprotein dieses Virus exprimiert, enthält und andererseits eine zweite Valenz umfaßt, die das antigene Hüllenglykoprotein dieses Virus enthält.

7. Polyvalentes Vakzin zur direkten Verwendung nach Anspruch 4, dadurch gekennzeichnet, daß die beiden Valenzen in Lösung in einem, wässrigen oder Ölartigen Lösungsmittel sind.

8. Polyvalentes Vakzin nach Anspruch 5, dadurch gekennzeichnet, daß sich die beiden; Valenzen in lyophilisierter Form und im Gemisch zur gleichzeitigen Aufnahme mit einem ölartigen oder wässrigen Lösungsmittel befinden.

9. Polyvalentes Vakzin nach Anspruch 5, dadurch gekennzeichnet, daß die erste Valenz lyophilisiert und die zweite flüssig ist, wobei die erste mit der zweiten aufgenommen werden muß.

10. Polyvalentes Vakzin nach Anspruch 5, dadurch gekennzeichnet, daß jede Valenz lyophilisiert ist und mit einem Lösungsmittel gleicher Natur wässrig oder ölig aufgenommen und dann mit der anderen Valenz vermischt wird.

11. Polyvalentes Vakzin nach Anspruch 6, dadurch gekennzeichnet, daß die beiden Valenzen lyophilisiert sind und getrennt voneinander mit einem wässrigen oder ölartigen Lösungsmittel aufgenommen werden müssen.

12. Polyvalentes Vakzin nach Anspruch 6, dadurch gekennzeichnet, daß die zweite Valenz flüssig und fertig zur Anwendung ist, wohingegen die erste lyophilisiert und durch ein wässriges oder ölartiges Lösungsmittel aufgenommen werden muß.

13. Polyvalentes Vakzin nach einem der Ansprüche 4 bis 12, dadurch gekennzeichnet, daß der betroffene tierische Virus ein Schweine-, Pferde-, Rinder-, Katzen- oder Affenherpesvirus ist.

14. Polyvalentes Vakzin nach Anspruch 13, dadurch gekennzeichnet, daß das betroffene Virus das Virus der Aujeszky-Krankheit ist.

15. Polyvalentes Vakzin nach einem der Ansprüche 1 bis 14, dadurch gekennzeichnet, daß sie in einer einzigen Umhüllung vorliegt.

## Patentansprüche (Patentansprüche für folgende(n) Vertragsstaat(en): ES, GR)

1. Verfahren zur Herstellung eines polyvalenten Vakzins gegen eine infektiöse Krankheit, dadurch gekennzeichnet, daß man in einer einzigen Umhüllung eins erste Valenz, die ein mikrobielles Mittel im lebend attenuierten Zustand oder einen Rekombinanten, welcher antigene Proteine oder Glykoproteine dieses Mittels exprimiert, enthält, und eine zweite Valenz, die ein oder mehrere antigene Hüllenglykoproteine oder Wände oder antigene Strukturproteine, inebesondere desselben Serotyps enthält, einbringt.

2. Verfahren nach Anspruch 1, daß das Mittel ausgewählt ist aus den Viren, den Bakterien und den Parasiten.

3. Verfahren nach Anspruch 2, dadurch gekennzeichnet, daß das Mittel von denjenigen ausgewählt ist, von denen man eine wenigstens teilweise schützende lebend attenuierte Form oder einen wenigstens teilweise schützenden Rekombinanten kennt.

4. Verfahren einem der Ansprüche 2 oder 3, die gegen einen Virus aus der Familie der tierischen oder menschlichen Herpesviren gerichtet ist und in einer einzigen Zubereitung eine erste Valenz umfaßt, die diesen Virus im lebend attenuierten Zustand oder einen Rekombinanten, welcher antigene Glykoproteine dieses Virus exprimiert, enthält, und eine zweite Valenz umfaßt, die antigene Hüllenglykoproteine dieses Virus enthält.

5. Verfahren nach Anspruch 4, dadurch gekennzeichnet, daß die beiden Valenzen miteinander vermischt werden.

6. Verfahren nach Anspruch 5, dadurch gekennzeichnet, daß die beiden Valenzen in Lösung in einem wässrigen oder ölartigen Lösungsmittel sind.

7. Verfahren nach Anspruch 4, dadurch gekennzeichnet, daß man die beiden Valenzen in lyophilisierter Form und in Mischung derart herstellt, daß sie unmittlebar vor Verwendung und gleichzeitig mit einem ölartigen oder wässrigen Lösungsmittel aufgenommen werden können.

8. Verfahren nach Anspruch 4, dadurch gekennzeichnet, daß man die erste Valenz in lyophilisierter Form und die zweite Valenz in flüssiger Form in eine Lösungsmittel herstellt, das befähigt ist, die erste Valenz aufzunehmen.

9. Verfahren nach Anspruch 4, dadurch gekennzeichnet, daß man jede Valenz in lyophilisierter Form herstellt, die mit einem Lösungsmittel derselben Natur wie derjenigen der bieden Valenzen, wässrig oder ölartig, aufgenommen und dann mit der anderen Valenz vermischt werden kann.

10. Verfahren nach Anspruch 4, dadurch gekennzeichnet, daß die beiden Valenzen jeweils in lyophilisierter Form derart hergestellt werden, daß sie getrennt voneinander mit einem wässrigen oder ölartigen Lösungsmittel aufgenommen werden können.

11. Verfahren nach Anspruch 4, dadurch gekennzeichnet, daß man die zweite Valenz in flüssiger Form und fertig zur Anwendung herstellt, wohingegen man die erste in lyophilisierter Form herstellt, die mit einem wässrigen oder ölartigen Lösungsmittel aufgenommen werde muß.

12. Verfahren nach einem der Ansprüche 4 bis 11, dadurch gekennzeichnet, daß der betroffene tierische Virus ein Schweine-, Pferde-, Rinder-, Katzen- oder Affenherpesvirus ist.

13. Verfahren nach Anspruch 12, dadurch gekennzeichnet, daß das betroffene Virus das Virus der Aujeszky-Krankheit ist.
